# EUROPÄISCHE PATENTSCHRIFT

(11) **EP 3 137 017 B1**
(45) Veröffentlichungstag und Bekanntmachung des Hinweises auf die Patenterteilung: **25.07.2018**
(21) Anmeldenummer: 15718907.7
(22) Anmeldetag: 29.04.2015
(51) Int. Cl.: A61F 2/38

(54) **KNIEGELENKENDOPROTHESE**
KNEE-JOINT ENDOPROSTHESIS
ENDOPROTHÈSE POUR L'ARTICULATION DU GENOU

(30) Priorität: 29.04.2014 DE 102014106012
(43) Veröffentlichungstag der Anmeldung: 08.03.2017
(73) Patentinhaber: Aesculap AG, 78532 Tuttlingen (DE)
(72) Erfinder: HAGEN, Thomas, 78532 Tuttlingen (DE); BÖTTIGER, Roland, 78604 Rietheim-Weilheim (DE); WILLMANN, Michael, 78048 Villingen-Schwenningen (DE); KEMPF, Harry, 72510 Stetten a. k. M. (DE)
(74) Vertreter: Hoeger, Stellrecht & Partner Patentanwälte mbB
(86) Internationale Anmeldenummer: PCT/EP2015/059321
(87) Internationale Veröffentlichungsnummer: WO 2015/165955

(56) Entgegenhaltungen:
- EP-A1- 2 213 262
- DE-A1- 2 122 390
- DE-A1- 4 102 509

## Beschreibung

Die vorliegende Erfindung betrifft eine Kniegelenkendoprothese mit einem Tibiateil, einem Femurteil, einen am Tibiateil drehfest angeordneten Meniskusteil und einer Verbindungseinrichtung zum gelenkigen Verbinden des Tibiateils mit dem Femurteil, welches Femurteil mindestens eine Femurkondyle mit einer Femurkondylenfläche aufweist, welches Meniskusteil mindestens eine dem Femurteil zugewandte Oberseite mit mindestens einer Meniskusgelenkfläche aufweist, welche die Femurkondylenfläche der mindestens einen Femurkondyle berührt, wobei das Femurteil und das Tibiateil relativ zueinander um eine Rotationsachse rotierbar gelagert sind, welche im Wesentlichen einer Längsachse der Tibia des Patienten entspricht, wobei das Tibiateil und das Femurteil relativ zueinander um eine Scharnierachse verschwenkbar gelagert sind, wobei die Scharnierachse quer zur Rotationsachse verläuft.

Kniegelenkendoprothesen der eingangs beschriebenen Art sind beispielsweise aus der DE 41 02 509 A1 bekannt. Es handelt sich dabei insbesondere um sogenannte Scharniergelenk-Knieprothesen, bei denen die Verbindungseinrichtung ein Scharniergelenk umfasst, um in ähnlicher Weise wie beim natürlichen Kniegelenk eine Flexion zwischen Unterschenkel und Oberschenkel zu ermöglichen. Weitere Scharniergelenk-Knieprothesen sind in der DE 2 122 390 A sowie in der EP 2 213 262 A1 beschrieben.

Allerdings ist die Kinematik des natürlichen Kniegelenks weitaus komplizierter als ein einfaches Scharniergelenk. Dies hat zur Folge, dass Patienten mit implantierten Kniegelenkendoprothesen der beschriebenen Art über eine eingeschränkte Beweglichkeit klagen.

Es ist daher eine Aufgabe der vorliegenden Erfindung, die Beweglichkeit einer Kniegelenkendoprothese der eingangs beschriebenen Art zu verbessern.

Diese Aufgabe wird bei einer Kniegelenkendoprothese der eingangs beschriebenen Art erfindungsgemäß dadurch gelöst, dass die Femurkondylenfläche der mindestens einen Femurkondyle und die mindestens eine Meniskusgelenkfläche korrespondierend zueinander ausgebildet sind derart, dass die Femurkondylenfläche der mindestens einen Femurkondyle einen Ausschnitt einer Kugeloberfläche oder im Wesentlichen einen Ausschnitt einer Kugeloberfläche definiert und dass die mindestens eine Meniskusgelenkfläche als eine an die Femurkondylenfläche angepasste Gelenkflächenausnehmung in Form einer gekrümmten Rinne ausgebildet ist.

Mit einer solchen Kniegelenkendoprothese ist es insbesondere möglich, das Tibiateil und das Femurteil nicht nur relativ zueinander um eine Scharnierachse zu verschwenken, sondern auch um eine Rotationsachse zu rotieren. Erfindungsgemäß entspricht die Rotationsachse im Wesentlichen einer Längsachse der Tibia des Patienten. So kann gleichzeitig bei einer Flexion des Knies auch eine mehr oder weniger stark ausgeprägte Rotation um eine Beinlängsachse des Patienten erfolgen. Um eine Verschleißanfälligkeit der Kniegelenkendoprothese, insbesondere von deren Meniskusteil, zu minimieren, ist es vorteilhaft, die mindestens eine Femurkondyle und die mindestens eine Meniskusgelenkfläche in der beschriebenen Weise auszubilden. So kann insbesondere ein linienförmiger Kontakt unabhängig von einer Flexions- oder Rotationsstellung des Tibiateils und des Femurteils relativ zueinander sichergestellt werden. Insgesamt kann somit insbesondere eine große Kongruenz zwischen dem auf dem Tibiateil feststehenden Meniskusteil und dem Femurteil erreicht werden. Die vorzugsweise von der Rotationsachse weg weisend konvex gekrümmte Gelenkflächenausnehmung kann sich zudem über eine vorgegebenen Rotationswinkelbereich erstrecken. Der Rotationswinkelbereich kann insbesondere patientenabhängig vorgegeben werden, um eine beschränkte Rotation des Femurteils und des Tibiateils relativ zueinander um die Rotationsachse zu gestatten. Beispielsweise kann so eine Rotation in einem Bereich von ±20° oder auch ±12° ermöglicht werden. Die gekrümmte Gelenkflächenausnehmung hat dann vorzugsweise eine Bogenlänge, die dem gewünschten Rotationswinkelbereich entspricht. Ein Querschnitt der Rinne quer zu ihrer Krümmung kann insbesondere kreisförmig oder auch elliptisch sein. Das Meniskusteil kann mit dem Tibiateil insbesondere durch eine Verdrehsicherung gegen eine Rotation gesichert werden. Alternativ oder zusätzlich können auch Schnapp-, Schraub- oder andere Verbindungsmechanismen vorgesehen sein, um das Meniskusteil drehfest oder vollständig unbeweglich am Tibiateil zu befestigen. Erfindungsgemäß sind das Tibiateil und das Femurteil relativ zueinander um eine Scharnierachse verschwenkbar gelagert. Die Scharnierachse ermöglicht es insbesondere auf einfache Weise, eine Beugebewegung zwischen dem Tibiateil und dem Femurteil zu gestatten, so dass die Kniegelenkendoprothese insgesamt eine annähernd natürliche Beugebewegung des Knies nachbilden kann. Günstig ist es, dass die Scharnierachse quer zur Rotationsachse verläuft. Die Rotationsachse kann zudem bezogen auf die Scharnierachse in anteriorer Richtung versetzt verlaufen, beispielsweise durch einen vorderen Bereich der Kniegelenkendoprothese. Diese Ausgestaltung hat insbesondere den Vorteil, dass eine Implantation der Kniegelenkendoprothese deutlich vereinfacht wird, da das Tibiateil und das Femurteil unabhängig voneinander so positioniert werden können, dass die miteinander zusammenwirkenden Elemente der Verbindungseinrichtung leicht zugänglich und miteinander in Eingriff bringbar sind.

Günstig ist es, wenn das Femurteil eine mediale und eine laterale Femurkondyle aufweist, welche eine mediale Femurkondylenfläche und eine laterale Femurkondylenfläche definieren, wenn das Meniskusteil eine mediale und eine laterale Meniskusgelenkfläche aufweist, wenn die mediale Femurkondylenfläche die mediale Meniskusgelenkfläche berührt und wenn die laterale Femurkondylenfläche die laterale Meniskusgelenkfläche berührt. In der beschriebenen Weise ist es insbesondere möglich, ein Femurteil mit zwei Femurkondylen auszustatten, so dass zwei Kontaktzonen oder Kontaktbereiche zwischen dem Femurteil und dem Meniskusteil definiert werden. Die Ausgestaltung der beiden Femurkondylenflächen in Form von Ausschnitten von Kugeloberflächen ermöglicht es insbesondere, sowohl lateralseitig als auch medialseitig mindestens einen linienförmigen Kontakt zwischen dem Femurteil und dem Meniskusteil zu erhalten.

Vorzugsweise weisen die mediale Femurkondylenfläche und die laterale Femurkondylenfläche einen identischen oder im Wesentlichen identischen Femurkondylenkrümmungsradius auf. So ist es insbesondere möglich, die Femurkondylen im Wesentlichen symmetrisch zu einer die Rotationsachse enthaltenden Symmetrieebene auszubilden. Entsprechend kann auch das Meniskusteil symmetrisch zu einer die Rotationsachse enthaltenden Symmetrieebene ausgebildet werden. Dies vereinfacht nicht nur die Konstruktion des Meniskusteils, sondern auch dessen Herstellung.

Vorteilhaft ist es ferner, wenn die mediale Femurkondylenfläche und die laterale Femurkondylenfläche voneinander abweichende Femurkondylenkrümmungsradien aufweisen. Dies gestattet es insbesondere, die Femurkondylen am Femurteil unterschiedlich groß auszubilden. Insbesondere kann so ein Femurteil ausgebildet werden, welches einem natürlichen Femur besonders nahe kommt. Beispielsweise kann diejenige Femurkondylenfläche mit einem größeren Femurkondylenkrümmungsradius ausgebildet werden, welche bei der Bewegung des Knies am stärksten belastet wird, um so einen möglichst großen Kontakt zwischen dem Femurteil und dem Meniskusteil zu erreichen, wodurch insbesondere ein Verschleiß, insbesondere des Meniskusteils, minimiert werden kann.

Günstig ist es ferner, wenn die mediale Meniskusgelenkfläche und die laterale Meniskusgelenkfläche einen identischen oder im Wesentlichen identischen Meniskusgelenkflächenkrümmungsradius aufweisen. Eine solche Ausgestaltung ist insbesondere dann vorteilhaft, wenn auch die mediale und laterale Femurkondylenfläche einen identischen oder im Wesentlichen identischen Femurkondylenkrümmungsradius aufweisen. So kann sowohl medialseitig als auch lateralseitig eine linienförmige oder im Wesentlichen linienförmige Anlage erreicht werden, und zwar abhängig von einem Flexions- und/oder Rotationswinkel zwischen Femurteil und Tibiateil.

Ferner kann es vorteilhaft sein, wenn die mediale Meniskusgelenkfläche und die laterale Meniskusgelenkfläche voneinander abweichende Meniskusgelenkflächenkrümmungsradien aufweisen. Insbesondere können die Meniskusgelenkflächenkrümmungsradien definiert werden als Krümmungsradien von Schnittlinien des Meniskusteils mit einer die Rotationsachse enthaltenden Schnittebene. Die Meniskusgelenkflächen sind aufgrund der korrespondierenden Ausbildung zu den Femurkondylenflächen vorzugsweise vom Meniskusteil weg weisend konkav gekrümmt.

Vorzugsweise ist die mindestens eine Meniskusgelenkfläche konzentrisch oder im Wesentlichen konzentrisch zur Rotationsachse ausgebildet. Insbesondere kann also die Gelenkflächenausnehmung in Form einer gekrümmten Rinne konzentrisch zur Rotationsachse ausgebildet sein. Auf diese Weise kann die mindestens eine Femurkondyle bei einer Rotation des Femurteils relativ zum Tibiateil in der rinnenförmigen Meniskusgelenkfläche geführt werden. So kann insbesondere unabhängig von einer Flexions- und Rotationsstellung des Femurteils und des Tibiateils relativ zueinander eine optimale, beispielsweise zumindest linienförmige Anlage der Femurkondylenfläche der mindestens einen Femurkondyle an der mindestens einen Meniskusgelenkfläche erreicht werden.

Gemäß einer weiteren bevorzugten Ausführungsform der Erfindung kann vorgesehen sein, dass die mindestens eine Meniskusgelenkfläche vom Meniskusteil weg weisend konkav gekrümmt ist und in einer beliebigen, die Rotationsachse enthaltenden Schnittebene einen Meniskusgelenkflächenkrümmungsradius aufweist, welcher einem Femurkondylenkrümmungsradius der vom Femurteil weg weisend konvex gekrümmten Femurkondylenfläche der mindestens einen Femurkondyle entspricht oder im Wesentlichen entspricht. Insbesondere bei einer Übereinstimmung des Meniskusgelenkflächenkrümmungsradius und des Femurkondylenkrümmungsradius kann so insbesondere eine linienförmige Anlage des Femurteils am Meniskusteil unabhängig von einer Rotations- und/oder Flexionsstellung von Femurteil und Tibiateil relativ zueinander sichergestellt werden.

Vorzugsweise sind die Meniskusgelenkflächenkrümmungsradien beliebiger, die Rotationsachse enthaltender Schnittebenen identisch oder im Wesentlichen identisch. So kann insbesondere unabhängig von einer Rotationsstellung eine linienförmige Anlage des Femurteils am Meniskusteil erreicht werden. Alternativ können die Krümmungsradien in Abhängigkeit einer Rotationswinkelstellung zwischen Femurteil und Tibiateil auch zu- oder abnehmen. Ferner kann sich auch eine Krümmung der Meniskusgelenkfläche in einer Schnittebene ändern. Beispielsweise kann so eine Schnittlinie des Meniskusteils mit der Schnittebene nicht zwingend einen Kreisbogenabschnitt bilden, sondern alternativ auch einen Ausschnitt einer Ellipse.

Damit die Kniegelenkendoprothese möglichst hohen Belastungen standhalten kann, ist es günstig, wenn ein Abstand zweier Mittelpunkte der die laterale Femurkondyle und die mediale Femurkondyle definierenden Kugelflächen kleiner ist als die Summe des lateralen Femurkondylenkrümmungsradius und des medialen Femurkondylenkrümmungsradius. Mit anderen Worten schneiden sich die von den beiden Femurkondylen definierten Kugelflächen. Je größer die Femurkondylenkrümmungsradien sind, umso länger sind die Berührlinien des Femurteils und des Meniskusteils. Je größer diese sind, umso geringer ein Verschleiß des vorzugsweise aus einem Kunststoff gebildeten Meniskusteils.

Vorteilhaft ist es, wenn Mittelpunkte von Schnittlinien zwischen der mindestens einen Meniskusgelenkfläche und beliebiger, die Rotationsachse enthaltender Schnittebenen auf einer zur Rotationsachse konzentrischen Zylinderfläche liegen. Damit weisen sie alle denselben Abstand von der Rotationsachse auf. Optional kann so aber auch die von der Meniskusgelenkfläche definierte Gelenkflächenausnehmung in ihrer Tiefe parallel zur Rotationsachse variieren. Mit anderen Worten müssen nicht alle Mittelpunkte zwingend auf einer Ebene liegen. Vielmehr können Positionen der Mittelpunkte in einer Richtung parallel zur Rotationsachse in Abhängigkeit eines Rotationswinkels der jeweiligen Schnittebene variieren. Beispielsweise kann so die Gelenkflächenausnehmung bei einer Rotationsstellung von 0° einen minimalen Abstand zwischen dem Femurteil und dem Tibiateil definieren, bei zunehmender Auslenkung jedoch einen größeren oder auch zunehmenden Abstand. Dies kann insbesondere helfen, das Knie mehr oder weniger automatisch wieder in seine neutrale Rotationsstellung zu überführen.

Günstigerweise liegen die Mittelpunkte auf einer Mittelpunktsebene, die quer, insbesondere senkrecht, zur Rotationsachse verläuft. Beispielsweise kann die Mittelpunktsebene parallel zu einer Tibiafläche verlaufen, an welcher das Meniskusteil anliegt. Optional kann die Mittelpunktsebene zu dieser Tibiafläche auch geneigt sein.

Besonders stabil ausbilden lässt sich die Kniegelenkendoprothese, wenn das Meniskusteil einstückig ausgebildet ist.

Vorteilhafterweise definiert die Verbindungseinrichtung eine Verbindungsstellung, in welcher das Tibiateil und das Femurteil unlösbar miteinander verbunden sind. Auf diese Weise kann insbesondere eine Luxation des Knies verhindert werden, also ein unbeabsichtigtes Lösen des Femurteils und des Tibiateils voneinander. Bei einer solchen Luxation besteht insbesondere die Gefahr, dass ein Kontakt zwischen dem Femurteil und dem Meniskusteil aufgehoben wird, so dass diese in undefinierter Weise aufeinander einwirken können, wobei insbesondere das Meniskusteil leicht beschädigt werden kann.

Günstig ist es, wenn das Tibiateil eine in Richtung auf das Meniskusteil hin weisende Meniskusteilanlagefläche aufweist und wenn die Rotationsachse quer, insbesondere senkrecht, zur Meniskusteilanlagefläche verläuft. Auf diese Weise ist es insbesondere möglich, vom Femurteil in Richtung einer vom Unterschenkel des Patienten definierten Längsachse Kräfte auf das Meniskusteil und über dieses auf das Tibiateil zu übertragen. Insbesondere dann, wenn das Tibiateil einen Schaft aufweist oder mit einem Schaft verbunden ist, welcher in einen Markraum der Tibia eingesetzt werden kann, können so Kräfte vom Femurteil in die Tibia abgeleitet werden.

Günstig ist es, wenn die Scharnierachse senkrecht zur Rotationsachse verläuft.

Gemäß einer weiteren bevorzugten Ausführungsform der Erfindung kann vorgesehen sein, dass das Tibiateil eine Tibiafläche aufweist, an welcher das Meniskusteil mit einer Meniskusteilunterseite anliegt, insbesondere flächig anliegt, und dass die Scharnierachse parallel oder im Wesentlichen parallel zur Tibiafläche verläuft. Diese Ausgestaltung ermöglicht es insbesondere, vom Femurteil auf das Meniskusteil wirkende Kräfte optimal in das Tibiateil abzuleiten. Ferner kann durch die besondere Gestaltung des Tibiateils ein Operateur bei der Implantation einfach und sicher nachvollziehen, ob die Kniegelenkendoprothese in gewünschter Weise mit dem verbliebenen Femur und dem verbliebenen Teil der Tibia verbunden ist.

Günstig ist es, wenn die Rotationsachse und die Scharnierachse relativ zueinander windschief verlaufen. Mit anderen Worten schneiden sie sich also nicht. Dies gestattet es insbesondere, die Rotationsachse relativ zur Scharnierachse in anteriorer Richtung zu versetzen, was das in Eingriff Bringen von die Verbindungseinrichtung ausbildenden Teilen und Elementen bei der Implantation der Kniegelenkendoprothese vereinfacht.

Vorteilhaft ist es, wenn die Rotationsachse zu einer die Scharnierachse enthaltenden Ebene senkrecht steht. Dies ermöglicht insbesondere eine Rotation des Femurteils und des Tibiateils einerseits um die Rotationsachse und andererseits wird eine Verschwenkung um die senkrecht hierzu verlaufende Scharnierachse ermöglicht.

Die Implantation der Kniegelenkendoprothese wird insbesondere dadurch vereinfacht, dass die Verbindungseinrichtung derart ausgebildet ist, dass das Tibiateil und das Femurteil nach einer Implantation an der Tibia und am Femur miteinander verbindbar sind. Es ist also auf diese Weise insbesondere möglich, das Tibiateil und das Femurteil unabhängig voneinander mit der Tibia und dem Femur eines Patienten zu verbinden und erst nach entsprechender Anpassung an die beiden Knochen miteinander zu verbinden.

Auf einfache Weise wird eine Rotation des Tibiateils und des Femurteils relativ zueinander um die Rotationsachse ermöglicht, wenn am Tibiateil ein Drehlagerelement angeordnet, ausgebildet oder gehalten ist zum rotierbaren Lagern des Femurteils um die Rotationsachse. Beispielsweise kann das Drehlagerelement in Form eines rotationssymmetrischen Zapfens ausgebildet sein.

Die Implantation der Kniegelenkendoprothese lässt sich insbesondere dadurch weiter vereinfachen, dass das Drehlagerelement um die Rotationsachse rotierbar am Tibiateil gelagert ist. Beispielsweise kann so in Abhängigkeit der Ausgestaltung des Drehlagerelements auch eine Stabilität der Kniegelenkendoprothese verbessert werden.

Vorteilhaft ist es, wenn das Tibiateil eine Drehlagerelementaufnahme umfasst, in welcher das Drehlagerelement gehalten ist. Auf diese Weise kann insbesondere eine stabile Verbindung zwischen dem Drehlagerelement und dem Tibiateil erreicht werden. Insbesondere kann das Drehlagerelement in der Drehlagerelementaufnahme des Tibiateils um die Rotationsachse rotierbar gelagert werden.

Zur Ausbildung einer besonders stabilen Kniegelenkendoprothese ist es günstig, wenn das Drehlagerelement in der Drehlagerelementaufnahme in einer Richtung parallel zur Rotationsachse unbeweglich gelagert ist. Auf diese Weise ist es nicht möglich, dass sich das Femurteil und das Tibiateil voneinander weg bewegen können. Insbesondere kann so eine Luxation der Kniegelenkendoprothese auf einfache Weise verhindert werden.

Ferner kann es günstig sein, wenn das Drehlagerelement in der Drehlagerelementaufnahme in einer Richtung parallel zur Rotationsachse verschiebbar gelagert ist. Eine solche Ausgestaltung ist insbesondere dann vorteilhaft, wenn eine gewisse Beweglichkeit des Femurteils und des Tibiateils relativ zueinander parallel zur Rotationsachse gewünscht wird. Insbesondere kann dies sogar erforderlich sein, wenn die Mittelpunkte der Meniskusgelenkflächenkrümmungsradien nicht auf einer Ebene liegen. In diesem Fall ist eine Beweglichkeit des Femurteils und des Tibiateils relativ zueinander parallel zur Rotationsachse sogar erforderlich, um ein Verklemmen des Femurteils und des Meniskusteils infolge einer Rotation des Femurteils und des Tibiateils relativ zueinander zu verhindern.

Gemäß einer weiteren bevorzugten Ausführungsform der Erfindung kann vorgesehen sein, dass die Kniegelenkendoprothese eine Anschlageinrichtung zum Begrenzen einer Bewegung des Drehlagerelements und der Drehlagerelementaufnahme relativ zueinander in distaler und/oder proximaler Richtung umfasst. Mit einer solchen Anschlageinrichtung kann insbesondere eine Bewegung des Femurteils und des Tibiateils aufeinander zu und/oder voneinander weg begrenzt werden. So kann zum einen insbesondere eine Luxationssicherung der Kniegelenkendoprothese erreicht werden und zum anderen eine Belastung auf das Meniskusteil begrenzt werden.

Um zu verhindern, dass das Drehlagerelement aus der Drehlagerelementaufnahme in unbeabsichtigter oder unerwünschter Weise austreten kann, ist es günstig, wenn die Kniegelenkendoprothese eine Sicherungseinrichtung zum Sichern des Drehlagerelements in der Drehlagerelementaufnahme umfasst.

Vorzugsweise umfasst die Kniegelenkendoprothese eine Lagerhülse zum Lagern des Drehlagerelements, welche Lagerhülse die Drehlagerelementaufnahme definiert. Eine solche Lagerhülse ermöglicht es insbesondere, das Drehlagerelement in einer Ausnehmung am Tibiateil anzuordnen, zu halten oder verschiebbar zu lagern, um so insbesondere wahlweise eine Bewegung der Teile relativ zueinander parallel zur Rotationsachse zu gestatten oder zu verhindern.

Ferner kann es günstig sein, wenn das Tibiateil eine Lagerhülsenaufnahme aufweist zu Aufnehmen der Lagerhülse. Die Lagerhülsenaufnahme ermöglicht es insbesondere, die Lagerhülse auf einer hinreichenden Länge parallel zur Rotationsachse am Tibiateil abzustützen.

Ferner ist es vorteilhaft, wenn die Sicherungseinrichtung ein Sicherungselement umfasst zum Sichern der Lagerhülse in der Lagerhülsenaufnahme. Eine solche Sicherungseinrichtung ermöglicht es insbesondere, eine Luxation der Kniegelenkendoprothese zu vermeiden.

Günstigerweise umfasst die Anschlageinrichtung einen Anschlag, welche eine Einführtiefe der Lagerhülse in die Lagerhülsenaufnahme begrenzt. Insbesondere kann so auf einfache Weise ein minimaler Abstand zwischen dem Femurteil und dem Tibiateil vorgegeben werden.

Vorteilhaft ist es, wenn die Drehlagerelementaufnahme in distaler Richtung geschlossen und in proximaler Richtung offen ist. Die Drehlagerelementaufnahme kann somit einerseits einen Anschlag bilden, um eine Bewegung des Femurteils und des Tibiateils aufeinander zu zu begrenzen. Andererseits wird es durch die offene Ausrichtung in proximaler Richtung ermöglicht, dass das Drehlagerelement aus der Drehlagerelementaufnahme herausragen kann, insbesondere auch über die oben beschriebene Tibiafläche vorstehen kann, um mit dem Femurteil direkt oder einem gelenkig an diesem um die Scharnierachse verschwenkbaren Verbindungselement in Eingriff gebracht zu werden.

Um eine Stabilität der Kniegelenkendoprothese zu verbessern, ist es insbesondere günstig, wenn sich die Drehlagerelementaufnahme mindestens teilweise oberhalb und/oder unterhalb der Tibiafläche erstreckt.

Um ein Schlagen von Metallteilen aneinander zu vermeiden, ist es günstig, wenn das Meniskusteil ganz oder teilweise aus einem Kunststoff hergestellt ist.

Insbesondere ist es vorteilhaft, wenn der Kunststoff Polyethylen (PE), Polyethylen mit ultrahohem Molekulargewicht (UHMWPE) oder Polyetheretherketon ist oder umfasst. Derartige Kunststoffe sind insbesondere als Implantatmaterialien zugelassen und weisen je nach Art der Herstellung eine gewünschte Abriebfestigkeit auf, um einen Verschleiß der Kniegelenkendoprothese zu minimieren.

Die nachfolgende Beschreibung bevorzugter Ausführungsformen der Erfindung dient im Zusammenhang mit den Zeichnungen der näheren Erläuterung. Es zeigen:
- Figur 1:: eine schematische perspektivische Explosionsdarstellung eines Ausführungsbeispiels einer Kniegelenkendoprothese;
- Figur 2:: eine teilweise geschnittene perspektivische Ansicht des am Tibiateil festgelegten Meniskusteils der Kniegelenkendoprothese aus Figur 1;
- Figur 3:: eine schematische, teilweise durchbrochene Gesamtansicht der montierten Kniegelenkendoprothese aus Figur 1 mit Femurteil, Meniskusteil und Tibiateil aus posteriorer Richtung;
- Figur 4:: eine teilweise durchbrochene Seitenansicht der Kniegelenkendoprothese aus Figur 1;
- Figur 5:: eine Draufsicht auf das Femurteil von posterior in einer Streckstellung der Kniegelenkendoprothese aus Figur 1;
- Figur 6:: eine teilweise durchbrochene Ansicht der Kniegelenkendoprothese aus Figur 1 von posterior, also in Richtung des Pfeils A in Figur 4 und in Richtung des Pfeils B in Figur 7;
- Figur 7:: eine teilweise durchbrochene Ansicht der Kniegelenkendoprothese aus Figur 1 in Draufsicht auf das Meniskusteil in Streckstellung; und
- Figur 8:: eine schematische Ansicht von zwei in einer konzentrisch zu einer Längsachse ausgebildeten Rinne geführten Kugeln.

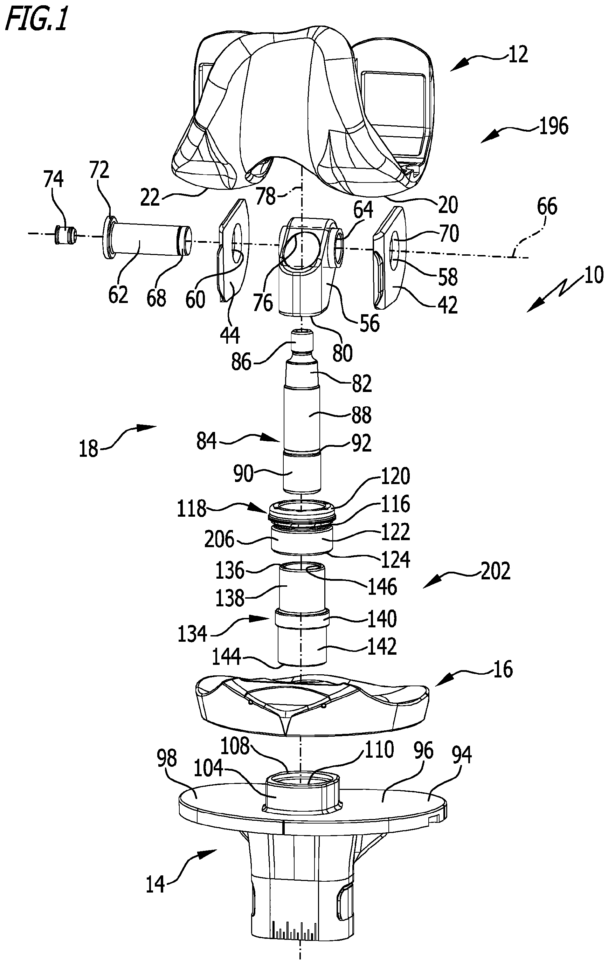

In Figur 1 ist beispielhaft eine Kniegelenkendoprothese mit ihren Einzelteilen dargestellt und insgesamt mit dem Bezugszeichen 10 bezeichnet. Sie umfasst ein Femurteil 12, welches mit oder ohne Schaft an einem Femur eines Patienten festgelegt werden kann, ein Tibiateil 14, welches an einer Tibia eines Patienten festgelegt werden kann und ein Meniskusteil 16, welches zwischen dem Femurteil 12 und dem Tibiateil 14 zumindest drehfest auf dem Tibiateil 14 angeordnet ist. Ferner umfasst die Kniegelenkendoprothese 10 eine Verbindungseinrichtung 18 umfassend mehrere Teile, mit welcher das Femurteil 12 und das Tibiateil 14 miteinander verbunden werden können.

Das Femurteil 12 umfasst eine mediale Femurkondyle 20 und eine laterale Femurkondyle 22. Diese bilden Gelenkflächen des Femurteils 12, und zwar eine mediale Femurkondylenfläche 24 und eine laterale Femurkondylenfläche 26.

Sowohl die mediale Femurkondylenfläche 24 als auch die laterale Femurkondylenfläche 26 bilden einen Ausschnitt einer Kugeloberfläche 28 beziehungsweise 30, welche Kugelmittelpunkte 32 und 34 definieren. Ein medialer Femurkondylenkrümmungsradius 36 der medialen Femurkondylenfläche 24 entspricht bei dem in den Figuren dargestellten Ausführungsbeispiel einem lateralen Femurkondylenkrümmungsradius 38 der lateralen Femurkondylenfläche 26. Ein Abstand 40 der Kugelmittelpunkte 32 und 24 ist kleiner als die Summe des medialen Femurkondylenkrümmungsradius 36 und des lateralen Femurkondylenkrümmungsradius 38.

Zwischen den beiden Femurkondylen 20 und 22 ist eine von parallel zueinander verlaufenden Seitenwänden 42 und 44 begrenzte Aussparung 46 ausgebildet. Diese ist zur Rückseite hin mit einer Deckwand 48 verschlossen.

Die Deckwand 48 weist eine Durchbrechung 50 auf, an die sich ein etwas schmalerer Schlitz 52 direkt anschließt. Der Schlitz 52 erstreckt sich im Wesentlichen in anterior-posteriorer Richtung. Die Seitenwände 42 und 44 bilden zusammen mit der Deckwand 48 eine Gelenkbox 54 für einen Gelenkzapfen 56.

Die Seitenwände 42 und 44 weisen jeweils eine Durchbrechung 58 beziehungsweise 60 auf, in die eine Gelenkwelle 62 eingesetzt ist. Der Gelenkzapfen 56 weist eine sich längs einer von ihm definierten Längsachse erstreckende Querbohrung 64 auf, die eine Scharnierachse 66 definiert und zur Aufnahme der Gelenkwelle 62 dient. Die Gelenkwelle 62 kann an ihrem einen Ende mit einem kurzen Außengewindeabschnitt 68 versehen werden, welcher zu einem Innengewinde 70, welches in der Durchbrechung 58 der Seitenwand 42 ausgebildet ist, korrespondiert. Insgesamt wird so ein Scharniergelenk 196 zwischen dem Femurteil 12 und dem Gelenkzapfen 56 ausgebildet.

Das andere Ende der Gelenkwelle 62 weist einen sich in radialer Richtung von der Scharnierachse 66 weg erstreckenden Ringflansch 72 auf, welcher in die an der Seitenwand 44 ausgebildete Aussparung formschlüssig eingreift. Das mit dem Ringflansch 72 versehene Ende der Gelenkwelle 62 weist ferner eine Werkzeugelementaufnahme auf, beispielsweise in Form eines Innenvielrunds, welche mit einem entsprechenden Verschlussstopfen 74 verschlossen wird.

In der beschriebenen Weise ist der Gelenkzapfen 56 in der Aussparung 46 um die Scharnierachse 66 verschwenkbar gelagert. Die Scharnierachse 66 verläuft parallel beziehungsweise im Wesentlichen parallel zu einer Verbindungslinie der Kugelmittelpunkte 32 und 34.

Am Gelenkzapfen 56 ist ferner eine Längsdurchbrechung 76 ausgebildet, die anterior bezogen auf die Querbohrung 64 verläuft. Sie definiert eine Rotationsachse 78 der Kniegelenkendoprothese 10.

Ausgehend von einem in Richtung auf das Meniskusteil 16 hin weisenden Ende 80 ist die Längsdurchbrechung 76 in Form eines Innenkonus ausgebildet, welcher zu einem konischen Abschnitt 82 eines rotationssymmetrisch ausgebildeten Drehlagerelements 84 korrespondiert.

An den konischen Abschnitt 82 des Drehlagerelements 84 schließt sich auf einer Seite ein im Außendurchmesser etwas verringerter Außengewindeabschnitt 86 an, auf welchen eine in den Figuren nicht dargestellte Sicherungsmutter aufgeschraubt werden kann, um eine vorzugsweise selbsthemmende Konusklemmung zwischen dem Drehlagerelement 84 und dem Gelenkzapfen 56 zu sichern. Idealerweise ist der Außengewindeabschnitt 86 so bemessen, dass er nicht aus Längsdurchbrechung 76 vorsteht.

An den konischen Abschnitt 82 schließt sich an dessen im Durchmesser dickeres Ende ein etwa dreimal so langer Zylinderabschnitt 88 an. In Richtung auf das Tibiateil 14 hin bildet ein weiterer Zylinderabschnitt 90 ein Ende des Drehlagerelements 84. Der Zylinderabschnitt 90 weist einen etwas größeren Außendurchmesser auf als der Zylinderabschnitt 88, so dass eine in Richtung auf das Femurteil 12 hin weisende, ringförmige Anschlagfläche 92 einer Anschlageinrichtung 200 zum Begrenzen einer Bewegung des Drehlagerelements 84 parallel zur Rotationsachse 78 ausgebildet wird.

Das Tibiateil 14 umfasst eine Platte 94 mit einer in Richtung auf das Femurteil 12 hin weisenden Oberseite 96, die eine ebene Tibiafläche 98 definiert. An einer Unterseite 100 der Platte 94 ist ein kurzer Schaftstutzen 102 ausgebildet, welcher optional mit einem in den Figuren nicht dargestellten Schaft in modularer Weise verlängert werden kann.

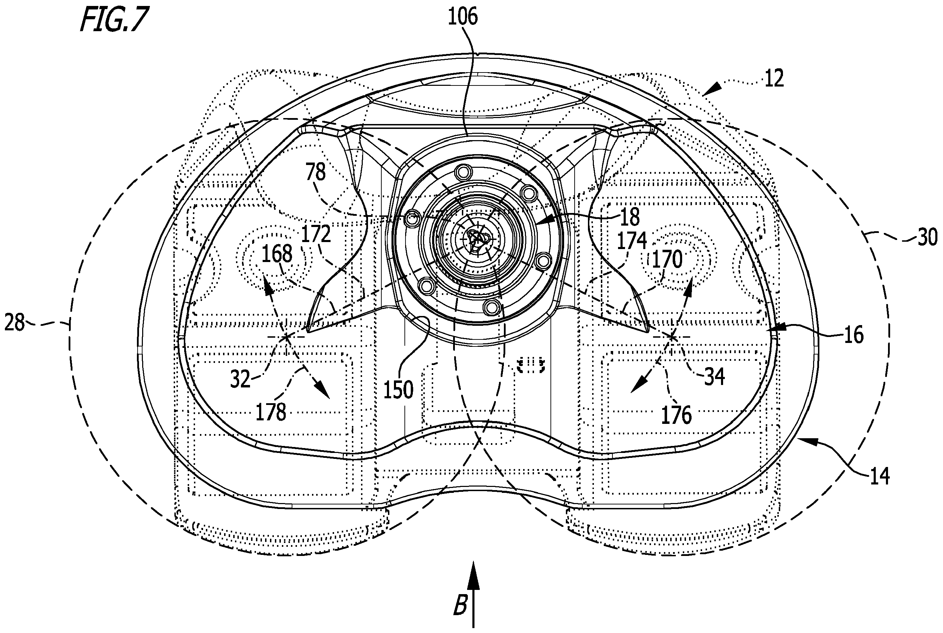

Von der Tibiafläche 98 erstreckt sich in Richtung auf das Femurteil 12 hin ein kurzer, hülsenförmiger Stutzen 104. Dieser weist eine unrunde Außenkontur 106 auf, wie insbesondere in Figur 7 gut zu erkennen.

Ausgehend von einem in Richtung auf das Femurteil 12 hin weisenden Ende 108 ist konzentrisch zur Rotationsachse 78 ein Sackloch 110 am Tibiateil 14 ausgebildet, welches einen in Richtung auf das Femurteil 12 hin weisenden Boden 112 aufweist. In Innern des Sacklochs 110 ist im Bereich des Stutzens 104 ein kurzer Innengewindeabschnitt 114 ausgebildet, welcher zu einem Au-ßengewindeabschnitt 116 einer Sicherungshülse 118 korrespondiert.

Die Sicherungshülse 118 weist ein ringflanschförmiges Ende 120 auf, dessen Außendurchmesser so bemessen ist, dass es einen Anschlag bildet, der am Ende 108 des Stutzens 104 anschlägt, wenn die Sicherungshülse 118 in den Stutzen 104 eingeschraubt ist. Eine Länge der Sicherungshülse 118 ist nur wesentlich größer als eine Höhe des sich von der Tibiafläche 98 erhebenden Stutzens 104.

An den Außengewindeabschnitt 116 schließt sich ein kurzer zylindrischer Hülsenabschnitt 122 an, dessen Außendurchmesser an einen Innendurchmesser des Sacklochs 110 im Bereich des Stutzens 104 angepasst ist.

Wenn die Sicherungshülse 118 in das Sackloch 110 eingeschraubt ist, reicht deren vom Femurteil 12 weg weisendes Ende 124 etwa bis zu einem ringförmigen Rücksprung 126 des Sacklochs 110 heran. Im Bereich des Rücksprungs 126 verringert sich ein Innendurchmesser des Sacklochs 110 etwas, so dass ein kurzer hohlzylindrischer Wandabschnitt 128 definiert wird. An den Wandabschnitt 128 schließt sich ein weiterer Rücksprung 130 an, in dessen Bereich sich ein Innendurchmesser des Sacklochs 110 nochmals verringert. In Richtung auf den Boden 112 hin ist das Sackloch 110 in Form einer hohlzylindrischen Ausnehmung 132 ausgebildet.

Zum gesicherten Verbinden des Femurteils 12 und des Tibiateils 14 umfasst die Verbindungseinrichtung 18 ferner eine rotationssymmetrisch zur Rotationsachse 78 ausgebildete Lagerhülse 134. Diese weist ausgehend von einem in Richtung auf das Femurteil 12 hin weisenden Ende 136 einen ersten Hülsenabschnitt 138 auf, an welchen sich ein im Außendurchmesser vergrößerter, kurzer zweiter Hülsenabschnitt 140 anschließt. An diesen wiederum schließt sich ein dritter Hülsenabschnitt 142 an, welcher sich bis zum anderen Ende 144 der Lagerhülse 134 erstreckt. Ein Außendurchmesser des dritten Hülsenabschnitts 142 ist etwas größer als ein Außendurchmesser des ersten Hülsenabschnitts 138, jedoch etwas kleiner als ein Außendurchmesser des zweiten Hülsenabschnitts 140.

Im Innern weist die Lagerhülse 134 eine rotationssymmetrisch ausgebildete Längsdurchbrechung 146 auf, die sich im Bereich des zweiten Hülsenabschnitts 140 in Richtung auf das Ende 144 hin einstufig im Innendurchmesser erweitert und eine Drehlagerelementaufnahme 198 definiert.

Das Meniskusteil 16 weist eine Meniskusteilunterseite 148 auf, die eben ist und an der eine Meniskusteilanlagefläche definierenden Tibiafläche 98 flächig anliegt. Ferner ist das Meniskusteil 16 mit einer Durchbrechung 150 versehen, deren Innenkontur 152 an die Außenkontur 106 des Stutzens 104 angepasst ist. Das Meniskusteil 16 kann auf diese Weise von oben auf das Tibiateil 14 aufgesetzt werden und ist aufgrund der unrunden Gestalt sowohl der Außenkontur 106 als auch der korrespondierend ausgebildeten Innenkontur 152 gegen eine Rotation, also drehfest, auf dem Tibiateil 14 gesichert.

Eine Oberseite 154 des Meniskusteils 16 weist eine mediale Meniskusgelenkfläche 156 und eine laterale Meniskusgelenkfläche 158 auf. Die Meniskusgelenkflächen 156 und 158 sind an die Femurkondylenflächen 24 und 26 angepasst und weisen mediale und laterale Meniskusgelenkflächenkrümmungsradien 192 und 194 auf, die vorzugsweise den Femurkondylenkrümmungsradien 36 und 38 entsprechen.

Die Meniskusgelenkflächen 156 und 158 definieren mediale und laterale Gelenkflächenausnehmungen 160 und 162, die in Form von kurzen gekrümmten und räumlich voneinander getrennten Rinnen 164 und 166 ausgebildet sind. Jede der beiden Rinnen 164 und 166 dient zur Lagerung und Führung einer der beiden Femurkondylen 20 beziehungsweise 22.

Die Rinnen 164 und 166 sind bezogen auf die Rotationsachse 78 von dieser weg weisend konvex gekrümmt und verlaufen konzentrisch um diese. Die Rinnen 164 und 166 definieren in einem Schnitt mit die Rotationsachse enthaltenden Schnittebenen 168 und 170 Kreisbogenabschnitte, deren Radien 172 und 174 den Femurkondylenkrümmungsradien 36 beziehungsweise 38 entsprechen. Die Rinnen 164 und 166 sind somit in Form von hohlzylindrischen Abschnitten auf der Oberseite 154 des Meniskusteils 16 ausgebildet, die konzentrisch zur Rotationsachse 78 verlaufen. Mittelpunkte der durch die Rinnen 164 und 166 mit den Schnittebenen 168 und 170 definierten Kreisbogenabschnitte fallen mit den Kugelmittelpunkten 32 und 34 der Femurkondylen 20 und 22 zusammen, wenn das Femurteil 12 mit den Femurkondylenflächen 24 und 26 an den Meniskusgelenkflächen 156 und 158 anliegt.

Die Rinnen 164 und 166 sind nicht eben, sondern weisen mindestens ein lokales Minimum auf, und zwar dergestalt, dass Symmetrieebenen der beiden Femurkondylen 20 und 22 einerseits sowie des Tibiateils 14 andererseits parallel zueinander ausgerichtet sind, optional auch zusammenfallen können, wenn sich die Femurkondylen 20 und 22 jeweils im lokalen Minimum der Rinnen 164 und 166 befinden. Von dieser Grundstellung ausgehend, die in Figur 7 schematisch dargestellt ist, kann nun das Femurteil 12 in Richtung der Pfeile 176 und 178 relativ zum Meniskusteil 16 und damit auch zum Tibiateil 14 in medialer beziehungsweise in lateraler Richtung um die Rotationsachse 78 verdreht werden beziehungsweise in einem eingeschränkten Winkelbereich rotiert werden, wenn die Kniegelenkendoprothese 10 montiert ist.

Ein Abstand der Meniskusgelenkflächen 156 und 158 beziehungsweise der Femurkondylenflächen 24 und 26 von der Meniskusteilunterseite 148 nimmt ausgehend von der Grundstellung vorzugsweise in medialer und lateraler Richtung zu, so dass sich infolge einer entsprechenden Rotation des Femurteils 12 und des Tibiateils 14 relativ zueinander das Femurteil 12 etwas vom Tibiateil 14 entfernt.

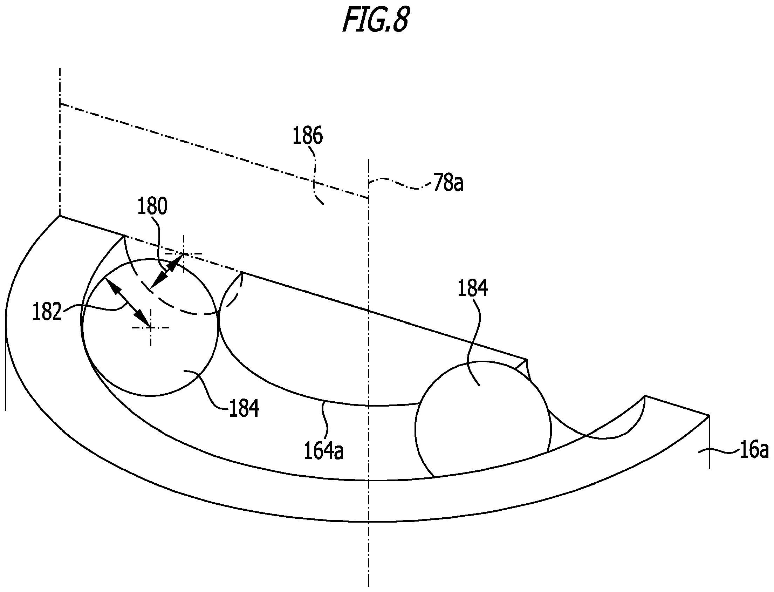

Das Zusammenwirken der Femurkondylen 20 und 22 mit den Meniskusgelenkflächen 156 und 168 des Meniskusteils 16 ist schematisch in Figur 8 dargestellt. Das in Figur 8 schematisch dargestellte Meniskusteil 16a weist eine Rinne 164a auf, die in Form einer hohlzylindrischen Ausnehmung ausgebildet ist und eine Gelenkflächenausnehmung 160a des Meniskusteils 16a bildet.

Ein Innenradius 180 der Rinne 164a entspricht einem Radius 182 der beiden in der Rinne 164a geführten Kugeln 184, die in dieser schematischen Darstellung Femurkondylen repräsentieren. Sind der Innenradius 180 und der Radius 182 identisch, ergibt sich eine linienförmige Anlage längs eines Kreisbogenabschnitts, welcher durch eine Schnittlinie zwischen einer die Rotationsachse 78a enthaltenden Schnittebene 186 mit dem Meniskusteil 16a definiert wird. Durch die konzentrische Ausrichtung der Rinne 164a zur Rotationsachse 78a sind beide Kugeln 184 optimal geführt und ermöglichen einen optimalen Kontakt, also insbesondere eine maximierte Kontaktfläche beziehungsweise -linie zwischen den Kugeln 184 und der Rinne 164a des Meniskusteils 16a.

Das Zusammenwirken der Femurkondylen 20 und 22 mit den Meniskusgelenkflächen 156 und 158 entspricht dem Zusammenwirken der Kugeln 184 und der Rinne 164a. Auf diese Weise ist es möglich, eine Kniegelenkendoprothese 10 auszubilden, die sowohl ein Scharniergelenk umfasst als auch eine Rotation des Femurteils 12 und des Tibiateils 14 um die Rotationsachse 78 ermöglicht, und das bei drehfest auf dem Tibialteil 14 angeordnetem Meniskusteil 16.

Bei der Implantation der Kniegelenkendoprothese 10 wird wie folgt verfahren. Das Verfahren ist kein Teil der Erfindung. Zunächst werden das Femurteil 12 und das Tibiateil 14 an die entsprechend präparierten Femur und Tibia des Patienten befestigt. Das Femurteil 12 ist dabei bereits gelenkig mit dem Gelenkzapfen 56 der Verbindungseinrichtung 18 in der oben beschriebenen Weise gekoppelt.

In einem nächsten Schritt kann das Meniskusteil 16 auf das Tibiateil 14 aufgesetzt werden.

Zum Verbinden des Femurteils 12 und des Tibiateils 14 miteinander wird zunächst das Drehlagerelement 84 mit dem Zylinderabschnitt 90 voran in das Sackloch 110 eingesetzt. Danach wird die Lagerhülse 134 mit ihrem Ende 144 voran über das Drehlagerelement 84 geschoben und in das Sackloch 110 eingesetzt, und zwar bis der zweite Hülsenabschnitt 140 am Rücksprung 130 anschlägt.

Als nächstes wird die Sicherungshülse 118, welche ein Sicherungselement 206 einer Sicherungseinrichtung 202 zum Sichern des Drehlagerelements 84 im eine Lagerhülsenaufnahme 204 definierenden Sackloch 100 bildet, mit ihrem Ende 124 voran in den Stutzen 104 eingesetzt und mit diesem verschraubt. Das Ende 124 der Sicherungshülse 118 liegt dann am zweiten Hülsenabschnitt 140 an und sichert diesen gegen eine Bewegung in axialer Richtung.

Das Drehlagerelement 84 ist in axialer Richtung etwas beweglich. Eine maximale Beweglichkeit desselben wird begrenzt durch die Anschlageinrichtung 200. Diese umfasst als einen Anschlag den Boden 112. Die maximale Beweglichkeit wird insbesondere definiert durch den Abstand des Bodens 112 und des Rücksprungs 130 im Innern der Lagerhülse 134, welcher als weiterer Anschlag der Anschlageinrichtung 200 für den Zylinderabschnitt 90 dient. Das Drehlagerelement 84 kann so einerseits am Boden 112 anschlagen und eine Bewegung des Femurteils 12 in Richtung auf das Tibiateil 14 begrenzen, zum anderen kann die Anschlagfläche 92 am Rücksprung im Innern der Lagerhülse 134 anschlagen und eine Bewegung des Femurteils 12 vom Tibiateil 14 weg begrenzen.

Wenn das Drehlagerelement 84 am Tibiateil 14 in der beschriebenen Weise gesichert ist, kann das Drehlagerelement 84 mit dem Außengewindeabschnitt 86 voran durch das Ende 80 der Längsdurchbrechung 76 am Gelenkzapfen 56 eingeführt werden. Der Gelenkzapfen 56 und das Drehlagerelement 84 werden über eine selbsthemmende Konusklemmung zwischen dem konischen Abschnitt 82 und dem korrespondierenden Innenkonusabschnitt am Gelenkzapfen 56 fest miteinander verbunden. Zur Sicherung der Konusklemmung kann wie beschrieben optional eine Sicherungsmutter auf den Außengewindeabschnitt 86 aufgeschraubt werden.

Die beschriebene Kniegelenkendoprothese 10 bildet ein sogenanntes "rotating hinge"-Knie mit feststehendem Meniskusteil 16, denn das Meniskusteil 16 kann relativ zum Tibiateil 14 wie erläutert nicht verdreht werden.

Die Verbindungseinrichtung 18 bildet eine Luxationssicherung, welche verhindert, dass das Femurteil 12 mit seinen Femurkondylen 20 und 22 aus den Meniskusgelenkflächen 156 und 158 des Meniskusteils 16 herausspringen kann.

Durch die besondere Formgestaltung der Femurkondylen 20 und 22 und der korrespondierenden Meniskusgelenkflächen 156 und 158 kann trotz der Rotationsmöglichkeit des Femurteils 12 und des Tibiateils 14 relativ zueinander um die Rotationsachse 78 ein Verschleiß minimiert werden.

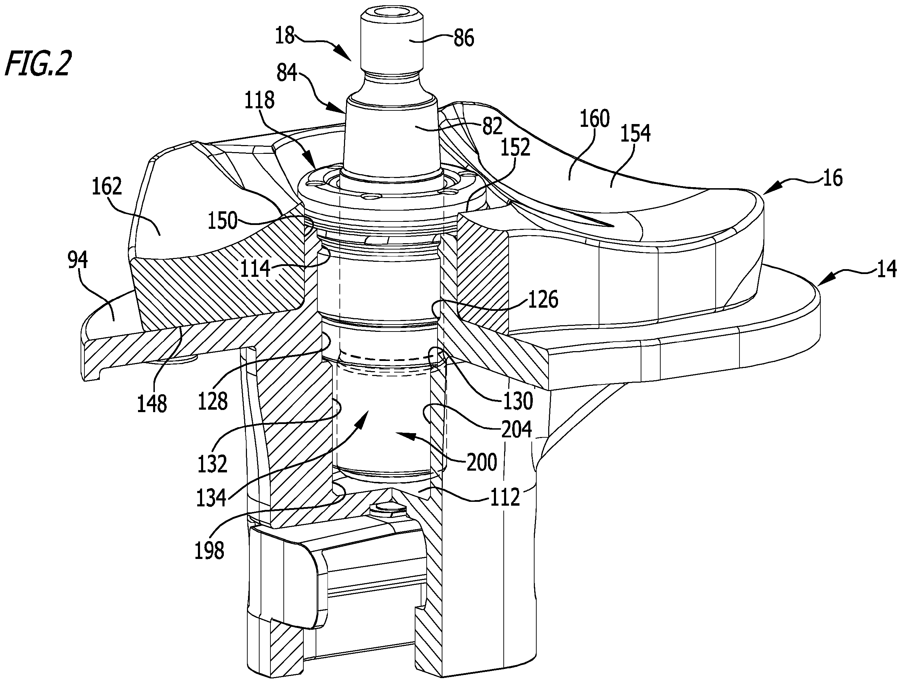

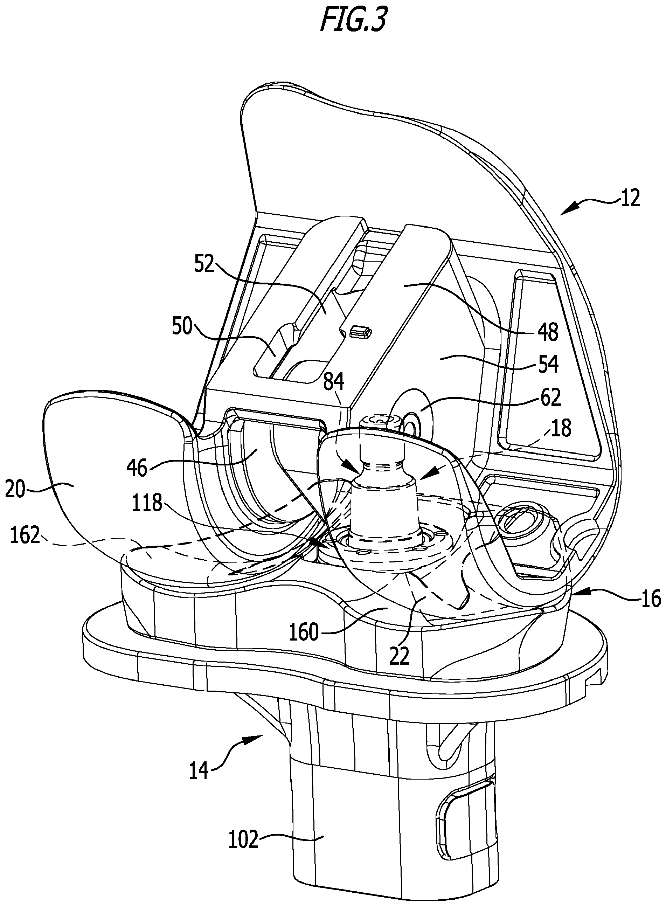

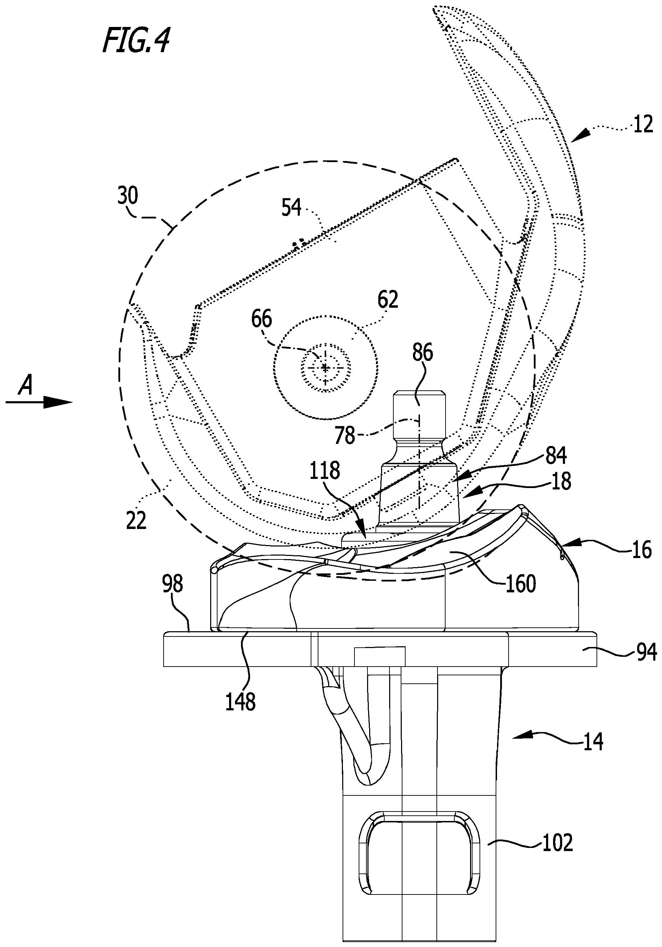

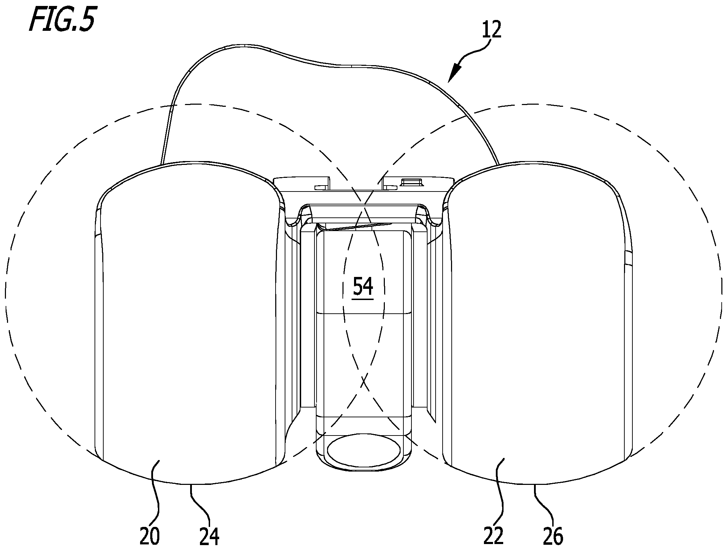

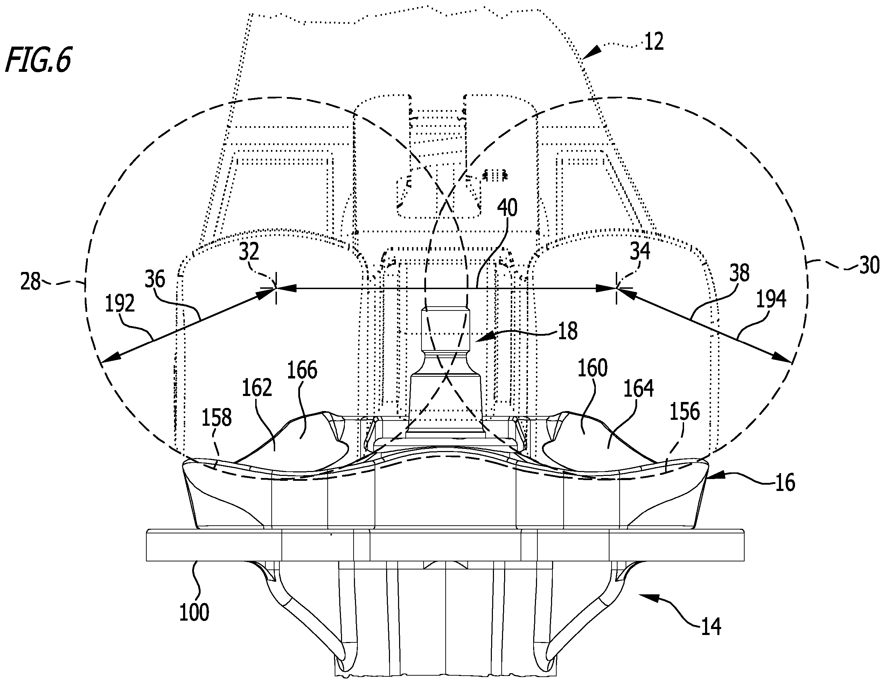

Die bei dem in den Figuren 1 bis 7 dargestellten Ausführungsbeispiel sich ändernde Tiefe der Rinne 164 beziehungsweise 166 bezogen auf die Meniskusteilunterseite 148 erhöht zudem bei einer Auslenkung des Femurteils 12 relativ zum Tibiateil 14 um die Rotationsachse 78 eine Bandspannung, so dass auch im Falle einer Rotation, beispielsweise in einem Winkelbereich von +-20°, insbesondere +-12°, um die Rotationsachse 78 eine zusätzliche Stabilisierung des implantierten Knies eintritt, wenn noch ein Teil des seitliche Bänder am Knie umfassenden Bandapparats des Patienten verbleibt.

Die Kniegelenkendoprothese 10 ist bis auf das Meniskusteil 16 vorzugsweise aus einem körperverträglichen Metall hergestellt. Sie kann teilweise aus einem Implantatstahl oder aber auch aus Titan oder einer Titanlegierung ausgebildet sein. Das Meniskusteil 16 kann insbesondere aus Polyethylen, insbesondere hochmolekulargewichtigem Polyethylen (UHMWPE), ausgebildet sein.

### Bezugszeichenliste

- 10: Kniegelenkendoprothese
- 12: Femurteil
- 14: Tibiateil
- 16: Meniskusteil
- 18: Verbindungseinrichtung
- 20: mediale Femurkondyle
- 22: laterale Femurkondyle
- 24: mediale Femurkondylenfläche
- 26: laterale Femurkondylenfläche
- 28: Kugeloberfläche
- 30: Kugeloberfläche
- 32: Kugelmittelpunkt
- 34: Kugelmittelpunkt
- 36: medialer Femurkondylenkrümmungsradius
- 38: lateraler Femurkondylenkrümmungsradius
- 40: Abstand
- 42: Seitenwand
- 44: Seitenwand
- 46: Aussparung
- 48: Deckwand
- 50: Durchbrechung
- 52: Schlitz
- 54: Gelenkbox
- 56: Gelenkzapfen
- 58: Durchbrechung
- 60: Durchbrechung
- 62: Gelenkwelle
- 64: Querbohrung
- 66: Scharnierachse
- 68: Außengewindeabschnitt
- 70: Innengewinde
- 72: Ringflansch
- 74: Verschlussstopfen
- 76: Längsdurchbrechung
- 78: Rotationsachse
- 80: Ende
- 82: konischer Abschnitt
- 84: Drehlagerelement
- 86: Außengewindeabschnitt
- 88: Zylinderabschnitt
- 90: Zylinderabschnitt
- 92: Anschlagfläche
- 94: Platte
- 96: Oberseite
- 98: Tibiafläche
- 100: Unterseite
- 102: Schaftstutzen
- 104: Stutzen
- 106: Außenkontur
- 108: Ende
- 110: Sackloch
- 112: Boden
- 114: Innengewindeabschnitt
- 116: Außengewindeabschnitt
- 118: Sicherungshülse
- 120: Ende
- 122: Hülsenabschnitt
- 124: Ende
- 126: Rücksprung
- 128: Wandabschnitt
- 130: Rücksprung
- 132: Ausnehmung
- 134: Lagerhülse
- 136: Ende
- 138: erster Hülsenabschnitt
- 140: zweiter Hülsenabschnitt
- 142: dritter Hülsenabschnitt
- 144: Ende
- 146: Längsdurchbrechung
- 148: Meniskusteilunterseite
- 150: Durchbrechung
- 152: Innenkontur
- 154: Oberseite
- 156: mediale Meniskusgelenkfläche
- 158: laterale Meniskusgelenkfläche
- 160: mediale Gelenkflächenausnehmung
- 162: laterale Gelenkflächenausnehmung
- 164: Rinne
- 166: Rinne
- 168: Schnittebene
- 170: Schnittebene
- 172: Radius
- 174: Radius
- 176: Pfeil
- 178: Pfeil
- 180: Innenradius
- 182: Radius
- 184: Kugel
- 186: Schnittebene
- 192: medialer Meniskusgelenkflächenkrümmungsradius
- 194: lateraler Meniskusgelenkflächenkrümmungsradius
- 196: Scharniergelenk
- 198: Drehlagerelementaufnahme
- 200: Anschlageinrichtung
- 202: Sicherungseinrichtung
- 204: Lagerhülsenaufnahme
- 206: Sicherungselement

## Patentansprüche

1. Kniegelenkendoprothese (10) mit einem Tibiateil (14), einem Femurteil (12), einem am Tibiateil (14) drehfest angeordneten Meniskusteil (16) und einer Verbindungseinrichtung (18) zum gelenkigen Verbinden des Tibiateils (14) mit dem Femurteil (12), welches Femurteil (12) mindestens eine Femurkondyle (20, 22) mit einer Femurkondylenfläche (24, 26) aufweist, welches Meniskusteil (16) mindestens eine dem Femurteil (12) zugewandte Oberseite (154) mit mindestens einer Meniskusgelenkfläche (156, 158) aufweist, welche die Femurkondylenfläche (24, 26) der mindestens einen Femurkondyle (20, 22) berührt, wobei das Femurteil (12) und das Tibiateil (14) relativ zueinander um eine Rotationsachse (78) rotierbar gelagert sind, welche im Wesentlichen einer Längsachse der Tibia des Patienten entspricht, wobei das Tibiateil (14) und das Femurteil (12) relativ zueinander um eine Scharnierachse (66) verschwenkbar gelagert sind, wobei die Scharnierachse (66) quer zur Rotationsachse (78) verläuft, **dadurch gekennzeichnet, dass** die Femurkondylenfläche (24, 26) der mindestens einen Femurkondyle (20, 22) und die mindestens eine Meniskusgelenkfläche (156, 158) korrespondierend zueinander ausgebildet sind derart, dass die Femurkondylenfläche (24, 26) der mindestens einen Femurkondyle (20, 22) einen Ausschnitt einer Kugeloberfläche (28, 30) oder im Wesentlichen einen Ausschnitt einer Kugeloberfläche (28, 30) definiert und dass die mindestens eine Meniskusgelenkfläche (156, 158) als eine an die Femurkondylenfläche (24, 26) angepasste Gelenkflächenausnehmung (160, 162) in Form einer gekrümmten Rinne (164, 166) ausgebildet ist.

2. Kniegelenkendoprothese nach Anspruch 1, **dadurch gekennzeichnet, dass** das Femurteil (12) eine mediale und eine laterale Femurkondyle (20, 22) aufweist, welche eine mediale Femurkondylenfläche (24) und eine laterale Femurkondylenfläche (26) definieren, dass das Meniskusteil (16) eine mediale und eine laterale Meniskusgelenkfläche (156, 158) aufweist, dass die mediale Femurkondylenfläche (24) die mediale Meniskusgelenkfläche (156) berührt und dass die laterale Femurkondylenfläche (26) die laterale Meniskusgelenkfläche (158) berührt.

3. Kniegelenkendoprothese nach Anspruch 2, **dadurch gekennzeichnet,**
a) **dass** die mediale Femurkondylenfläche (24) und die laterale Femurkondylenfläche (26) einen identischen oder im Wesentlichen identischen Femurkondylenkrümmungsradius (36, 38) aufweisen
oder
**dass** die mediale Femurkondylenfläche (24) und die laterale Femurkondylenfläche (26) voneinander abweichende Femurkondylenkrümmungsradien (36, 38) aufweisen
und/oder
b) **dass** die mediale Meniskusgelenkfläche (156) und die laterale Meniskusgelenkfläche (158) einen identischen oder im Wesentlichen identischen Meniskusgelenkflächenkrümmungsradius (192, 194) aufweisen
oder
**dass** die mediale Meniskusgelenkfläche (156) und die laterale Meniskusgelenkfläche (158) voneinander abweichende Meniskusgelenkflächenkrümmungsradien (192, 194) aufweisen.

4. Kniegelenkendoprothese nach einem der voranstehenden Ansprüche, **dadurch gekennzeichnet, dass** die mindestens eine Meniskusgelenkfläche (156, 158) konzentrisch oder im Wesentlichen konzentrisch zur Rotationsachse (78) ausgebildet ist.

5. Kniegelenkendoprothese nach einem der voranstehenden Ansprüche, **dadurch gekennzeichnet, dass** die mindestens eine Meniskusgelenkfläche (156, 158) vom Meniskusteil (16) weg weisend konkav gekrümmt ist und in einer beliebigen, die Rotationsachse (78) enthaltenden Schnittebene einen Meniskusgelenkflächenkrümmungsradius (192, 194) aufweist, welcher einem Femurkondylenkrümmungsradius (36, 38) der vom Femurteil (12) weg weisend konvex gekrümmten Femurkondylenfläche (24, 26) der mindestens einen Femurkondyle (20, 22) entspricht oder im Wesentlichen entspricht.

6. Kniegelenkendoprothese nach Anspruch 5, **dadurch gekennzeichnet,**
a) **dass** die Meniskusgelenkflächenkrümmungsradien (192, 194) beliebiger, die Rotationsachse (78) enthaltender Schnittebenen identisch oder im Wesentlichen identisch sind
und/oder
b) **dass** ein Abstand (40) zweier Mittelpunkte (32, 34) der die laterale Femurkondyle (22) und die mediale Femurkondyle (20) definierender Kugelflächen (28, 30) kleiner ist als die Summe des lateralen Femurkondylenkrümmungsradius (38) und des medialen Femurkondylenkrümmungsradius (36).

7. Kniegelenkendoprothese nach einem der voranstehenden Ansprüche, **dadurch gekennzeichnet,**
a) **dass** Mittelpunkte (32, 34) von Schnittlinien zwischen der mindestens einen Meniskusgelenkfläche (156, 158) und beliebiger, die Rotationsachse (78) enthaltender Schnittebenen auf einer zur Rotationsachse (78) konzentrischen Zylinderfläche liegen
und/oder
b) **dass** die Mittelpunkte (32, 34) auf einer Mittelpunktsebene liegen, die quer, insbesondere senkrecht, zur Rotationsachse (78) verläuft
und/oder
c) **dass** das Meniskusteil (16) einstückig ausgebildet ist
und/oder
d) **dass** die Verbindungseinrichtung (18) eine Verbindungsstellung definiert, in welcher das Tibiateil (14) und das Femurteil (12) unlösbar miteinander verbunden sind
und/oder
e) **dass** das Tibiateil (14) eine in Richtung auf das Meniskusteil (16) hin weisende Meniskusteilanlagefläche (98) aufweist und dass die Rotationsachse (78) quer, insbesondere senkrecht, zur Meniskusteilanlagefläche (98) verläuft.

8. Kniegelenkendoprothese nach einem der voranstehenden Ansprüche, **dadurch gekennzeichnet, dass**
a) die Scharnierachse (66) senkrecht zur Rotationsachse (78) verläuft und/oder
b) das Tibiateil (14) eine Tibiafläche (98) aufweist, an welcher das Meniskusteil (16) mit einer Meniskusteilunterseite (148) anliegt, insbesondere flächig, und dass die Scharnierachse (66) parallel oder im Wesentlichen parallel zur Tibiafläche (98) verläuft.

9. Kniegelenkendoprothese nach einem der voranstehenden Ansprüche, **dadurch gekennzeichnet,**
a) **dass** die Rotationsachse (78) und die Scharnierachse (66) relativ zueinander windschief verlaufen
und/oder
b) **dass** die Rotationsachse (78) zu einer die Scharnierachse (66) enthaltenden Ebene senkrecht steht
und/oder
c) **dass** die Verbindungseinrichtung (18) derart ausgebildet ist, dass das Tibiateil (14) und das Femurteil (12) nach einer Implantation an der Tibia und am Femur miteinander verbindbar sind,
wobei insbesondere am Tibiateil (14) ein Drehlagerelement (84) angeordnet, ausgebildet oder gehalten ist zum rotierbaren Lagern des Femurteils (12) um die Rotationsachse (78).

10. Kniegelenkendoprothese nach Anspruch 9, **dadurch gekennzeichnet,**
a) **dass** das Drehlagerelement (84) um die Rotationsachse (78) rotierbar am Tibiateil (14) gelagert ist
und/oder
b) **dass** das Tibiateil (14) eine Drehlagerelementaufnahme (198) umfasst, in welcher das Drehlagerelement (84) gehalten ist.

11. Kniegelenkendoprothese nach Anspruch 10, **dadurch gekennzeichnet,**
a) **dass** das Drehlagerelement (84) in der Drehlagerelementaufnahme (198) in einer Richtung parallel zur Rotationsachse (78) unbeweglich gelagert ist
oder
**dass** das Drehlagerelement (84) in der Drehlagerelementaufnahme (198) in einer Richtung parallel zur Rotationsachse (78) verschiebbar gelagert ist
und/oder
b) **dass** die Kniegelenkendoprothese eine Anschlageinrichtung (200) zum Begrenzen einer Bewegung des Drehlagerelements (84) und der Drehlagerelementaufnahme (198) relativ zueinander in distaler und/oder proximaler Richtung umfasst
und/oder
c) **dass** die Kniegelenkendoprothese eine Sicherungseinrichtung (202) zum Sichern des Drehlagerelements (84) in der Drehlagerelementaufnahme (198) umfasst
und/oder
d) **dass** die Kniegelenkendoprothese eine Lagerhülse (134) zum Lagern des Drehlagerelements (84) umfasst, welche die Drehlagerelementaufnahme (198) definiert.

12. Kniegelenkendoprothese nach Anspruch 11, **dadurch gekennzeichnet, dass** das Tibiateil (14) eine Lagerhülsenaufnahme (204) aufweist zum Aufnehmen der Lagerhülse (134).

13. Kniegelenkendoprothese nach Anspruch 12, **dadurch gekennzeichnet,**
a) **dass** die Sicherungseinrichtung (202) ein Sicherungselement (206) umfasst zum Sichern der Lagerhülse (134) in der Lagerhülsenaufnahme (204)
und/oder
b) **dass** die Anschlageinrichtung (200) einen Anschlag umfasst, welcher eine Einführtiefe der Lagerhülse (134) in die Lagerhülsenaufnahme (204) begrenzt.

14. Kniegelenkendoprothese nach einem der Ansprüche 10 bis 13, **dadurch gekennzeichnet,**
a) **dass** die Drehlagerelementaufnahme (198) in distaler Richtung geschlossen und in proximaler Richtung offen ist
und/oder
b) **dass** sich die Drehlagerelementaufnahme (198) mindestens teilweise oberhalb und/oder unterhalb der Tibiafläche (98) erstreckt.

15. Kniegelenkendoprothese nach einem der voranstehenden Ansprüche, **dadurch gekennzeichnet, dass** das Meniskusteil (16) ganz oder teilweise aus einem Kunststoff hergestellt ist,
wobei insbesondere der Kunststoff Polyethylen (PE), Polyethylen mit ultrahohem Molekulargewicht (UHMWPE) oder Polyetheretherketon ist oder umfasst.

## Claims

1. Knee joint endoprosthesis (10) having a tibia part (14), a femur part (12), a meniscus part (16) arranged on the tibia part (14) in mutually non-rotatable manner and a connecting device (18) for connecting the tibia part (14) to the femur part (12) in articulated manner, which femur part (12) comprises at least one femur condyle (20, 22) having a femur condyle surface (24, 26), which meniscus part (16) comprises at least one upper surface (154) which faces the femur part (12) and has at least one meniscus joint surface (156, 158) which touches the femur condyle surface (24, 26) of the at least one femur condyle (20, 22), wherein the femur part (12) and the tibia part (14) are mounted such as to be rotatable relative to each other about a rotational axis (78), which corresponds substantially to a longitudinal axis of the tibia of the patient, wherein the tibia part (14) and the femur part (12) are mounted such that they are pivotal relative to each other about a hinge axis (66), wherein the hinge axis (66) runs transversely to the rotational axis (78), **characterized in that** the femur condyle surface (24, 26) of the at least one femur condyle (20, 22) and the at least one meniscus joint surface (156, 158) are formed in correspondence with one another in such a manner that the femur condyle surface (24, 26) of the at least one femur condyle (20, 22) defines a section of a spherical surface (28, 30) or substantially a section of a spherical surface (28, 30) and that the at least one meniscus joint surface (156, 158) is formed as a joint surface recess (160, 162) in the form of a curved hollow (164, 166) which is matched to the femur condyle surface (24, 26).

2. A knee joint endoprosthesis in accordance with claim 1, **characterized in that** the femur part (12) comprises a medial and a lateral femur condyle (20, 22) which define a medial femur condyle surface (24) and a lateral femur condyle surface (26), **in that** the meniscus part (16) comprises a medial and a lateral meniscus joint surface (156, 158), **in that** the medial femur condyle surface (24) touches the medial meniscus joint surface (156) and **in that** the lateral femur condyle surface (26) touches the lateral meniscus joint surface (158).

3. A knee joint endoprosthesis in accordance with claim 2, **characterized**
a) **in that** the medial femur condyle surface (24) and the lateral femur condyle surface (26) have an identical or substantially identical femur condyle radius of curvature (36, 38)
or
**in that** the medial femur condyle surface (24) and the lateral femur condyle surface (26) have femur condyle radii of curvature (36, 38) which differ from each other.
and/or
b) **in that** the medial meniscus joint surface (156) and the lateral meniscus joint surface (158) have an identical or substantially identical meniscus joint surface radius of curvature (192, 194)
or
**in that** the medial meniscus joint surface (156) and the lateral meniscus joint surface (158) have meniscus joint surface radii of curvature (192, 194) which differ from each other.

4. A knee joint endoprosthesis in accordance with any one of the preceding claims, **characterized in that** the at least one meniscus joint surface (156, 158) is formed concentrically or substantially concentrically with respect to the rotational axis (78).

5. A knee joint endoprosthesis in accordance with any one of the preceding claims, **characterized in that** the at least one meniscus joint surface (156, 158) is concavely curved in a direction pointing away from the meniscus part (16) and has a meniscus joint surface radius of curvature (192, 194) in an arbitrary cutting plane containing the rotational axis (78) which corresponds or substantially corresponds to a femur condyle radius of curvature (36, 38) of the femur condyle surface (24, 26) of the at least one femur condyle (20, 22) which is convexly curved in a direction pointing away from the femur part (12).

6. A knee joint endoprosthesis in accordance with claim 5, **characterized**
a) **in that** the meniscus joint surface radii of curvature (192, 194) of arbitrary cutting planes containing the rotational axis (78) are identical or substantially identical
and/or
b) **in that** a spacing (40) of two centre points (32, 34) of the spherical surfaces (28, 30) defining the lateral femur condyle (22) and the medial femur condyle (20) is smaller than the sum of the lateral femur condyle radius of curvature (38) and the medial femur condyle radius of curvature (36).

7. A knee joint endoprosthesis in accordance with any one of the preceding claims, **characterized**
a) **in that** centre points (32, 34) of lines of intersection between the at least one meniscus joint surface (156, 158) and arbitrary cutting planes containing the rotational axis (78) are located on a cylinder surface that is concentric with the rotational axis (78),
and/or
b) **in that** the centre points (32, 34) lie on a centre point plane which runs transversely, in particular perpendicularly, to the rotational axis (78)
and/or
c) **in that** the meniscus part (16) is formed in one piece
and/or
d) **in that** the connecting device (18) defines a connecting position in which the tibia part (14) and the femur part (12) are connected to one another inseparably
and/or
e) **in that** the tibia part (14) comprises a meniscus part contact surface (98) pointing in the direction of the meniscus part (16) and in that the rotational axis (78) runs transversely, in particular perpendicularly, to the meniscus part contact surface (98).

8. A knee joint endoprosthesis in accordance with any one of the preceding claims, **characterized**
a) **in that** the hinge axis (66) runs perpendicularly to the rotational axis (78)
and/or
b) **in that** the tibia part (14) comprises a tibia surface (98) on which a meniscus part lower surface (148) of the meniscus part (16) rests, in particular in a planar manner, and in that the hinge axis (66) runs parallel or substantially parallel to the tibia surface (98).

9. A knee joint endoprosthesis in accordance with any one of the preceding claims, **characterized**
a) **in that** the rotational axis (78) and the hinge axis (66) are skewed relative to each other
and/or
b) **in that** the rotational axis (78) is perpendicular to a plane containing the hinge axis (66)
and/or
c) **in that** the connecting device (18) is formed in such a manner that the tibia part (14) and the femur part (12) are connectable to one another after an implantation on the tibia and on the femur, wherein, in particular, a rotary bearing element (84) is arranged, formed or held on the tibia part (14) for mounting the femur part (12) so as to be rotatable about the rotational axis (78).

10. A knee joint endoprosthesis in accordance with claim 9, **characterized**
a) **in that** the rotary bearing element (84) is mounted on the tibia part (14) such as to be rotatable about the rotational axis (78)
and/or
b) **in that** the tibia part (14) comprises a rotary bearing element seating (198) in which the rotary bearing element (84) is held.

11. A knee joint endoprosthesis in accordance with claim 10, **characterized**
a) **in that** the rotary bearing element (84) is mounted in the rotary bearing element seating (198) such as to be immovable in a direction parallel to the rotational axis (78)
or
**in that** the rotary bearing element (84) is mounted in the rotary bearing element seating (198) such as to be displaceable in a direction parallel to the rotational axis (78)
and/or
b) **in that** the knee joint endoprosthesis comprises a stop device (200) for limiting a movement of the rotary bearing element (84) and the rotary bearing element seating (198) relative to each other in the distal and/or proximal direction
and/or
c) **in that** the knee joint endoprosthesis comprises a securing device (202) for securing the rotary bearing element (84) in the rotary bearing element seating (198)
and/or
d) **in that** the knee joint endoprosthesis comprises a bearing sleeve (134) which defines the rotary bearing element seating (198) for mounting the rotary bearing element (84).

12. A knee joint endoprosthesis in accordance with claim 11, **characterized in that** the tibia part (14) comprises a bearing sleeve seating (204) for accommodating the bearing sleeve (134).

13. A knee joint endoprosthesis in accordance with claim 12, **characterized**
a) **in that** the securing device (202) comprises a securing element (206) for securing the bearing sleeve (134) in the bearing sleeve seating (204)
and/or
b) **in that** the stop device (200) comprises a stop which limits a depth of insertion of the bearing sleeve (134) into the bearing sleeve seating (204).

14. A knee joint endoprosthesis in accordance with any one of claims 10 to 13, **characterized**
a) **in that** the rotary bearing element seating (198) is closed in the distal direction and open in the proximal direction
and/or
b) **in that** the rotary bearing element seating (198) extends at least partly above and/or below the tibia surface (98).

15. A knee joint endoprosthesis in accordance with any one of the preceding claims, **characterized in that** the meniscus part (16) is made entirely or partly of a plastics material,
in particular, wherein the plastics material is or comprises polyethylene (PE), polyethylene of ultrahigh molecular weight (UHMWPE) or polyetheretherketone.

## Revendications

1. Endoprothèse de l'articulation du genou (10) avec une partie tibia (14), une partie fémur (12), une partie ménisque (16) disposée de manière solidaire en rotation sur la partie tibia (14) et un dispositif de liaison (18) pour la liaison articulée de la partie tibia (14) avec la partie fémur (12), laquelle partie fémur (12) présente au moins un condyle fémoral (20, 22) avec une surface de condyle fémoral (24, 26), laquelle partie ménisque (16) présente au moins un côté supérieur (154) tourné vers la partie fémur (12) avec au moins une surface d'articulation de ménisque (156, 158), qui touche la surface de condyle fémoral (24, 26) du au moins un condyle fémoral (20, 22), où la partie fémur (12) et la partie tibia (14) sont montées de manière à pouvoir tourner l'une par rapport à l'autre autour d'un axe de rotation (78) qui correspond sensiblement à un axe longitudinal du tibia du patient, où la partie tibia (14) et la partie fémur (12) sont montées de manière à pouvoir pivoter l'une par rapport à l'autre autour d'un axe de charnière (66), où l'axe de charnière (66) s'étend transversalement à l'axe de rotation (78), **caractérisée en ce que** la surface de condyle fémoral (24, 26) du au moins un condyle fémoral (20, 22) et la au moins une surface d'articulation de ménisque (156, 158) sont agencées en correspondance l'une avec l'autre de telle manière que la surface de condyle fémoral (24, 26) du au moins un condyle fémoral (20, 22) définit un secteur d'une surface sphérique (28, 30) ou sensiblement un secteur d'une surface sphérique (28, 30) et **en ce que** la au moins une surface d'articulation de ménisque (156, 158) est agencée comme un logement de surface d'articulation (160, 162) adapté à la surface de condyle fémoral (24, 26) sous forme d'une rigole courbée (164, 166).

2. Endoprothèse de l'articulation du genou selon la revendication 1, **caractérisée en ce que** la partie fémur (12) présente un condyle fémoral médian et un condyle fémoral latéral (20, 22) qui définissent une surface de condyle fémoral médiane (24) et une surface de condyle fémoral latérale (26), **en ce que** la partie ménisque (16) présente une surface d'articulation de ménisque médiane et une surface d'articulation de ménisque latérale (156, 158), **en ce que** la surface de condyle fémoral médiane (24) touche la surface d'articulation de ménisque médiane (156) et **en ce que** la surface de condyle fémoral latérale (26) touche la surface d'articulation de ménisque latérale (158).

3. Endoprothèse de l'articulation du genou selon la revendication 2, **caractérisée**
a) **en ce que** la surface de condyle fémoral médiane (24) et la surface de condyle fémoral latérale (26) présentent un rayon de courbure de condyle fémoral (36, 38) identique ou sensiblement identique
ou
**en ce que** la surface de condyle fémoral médiane (24) et la surface de condyle fémoral latérale (26) présentent des rayons de courbure de condyle fémoral (36, 38) différents l'un de l'autre
et/ou
b) **en ce que** la surface d'articulation de ménisque médiane (156) et la surface d'articulation de ménisque latérale (158) présentent un rayon de courbure de surface d'articulation de ménisque (192, 194) identique ou sensiblement identique
ou
**en ce que** la surface d'articulation de ménisque médiane (156) et la surface d'articulation de ménisque latérale (158) présentent des rayons de courbure de surface d'articulation de ménisque (192, 194) différents l'un de l'autre.

4. Endoprothèse de l'articulation du genou selon l'une des revendications précédentes, **caractérisée en ce que** la au moins une surface d'articulation de ménisque (156, 158) est agencée de manière concentrique ou sensiblement concentrique à l'axe de rotation (78).

5. Endoprothèse de l'articulation du genou selon l'une des revendications précédentes, **caractérisée en ce que** la au moins une surface d'articulation de ménisque (156, 158) est courbée de manière concave en étant tournée vers l'opposé de la partie ménisque (16) et présente dans un plan d'intersection quelconque contenant l'axe de rotation (78) un rayon de courbure de surface d'articulation de ménisque (192, 194) qui correspond ou correspond sensiblement à un rayon de courbure de condyle fémoral (36, 38) de la surface de condyle fémoral (24, 26) du au moins un condyle fémoral (20, 22) courbée de manière convexe en étant tournée vers l'opposé de la partie fémur (12).

6. Endoprothèse de l'articulation du genou selon la revendication 5, **caractérisée**
a) **en ce que** les rayons de courbure de surface d'articulation de ménisque (192, 194) de plans d'intersection quelconques contenant l'axe de rotation (78) sont identiques ou sensiblement identiques
et/ou
b) **en ce qu'**une distance (40) de deux points centraux (32, 34) des surfaces sphériques (28, 30) définissant le condyle fémoral latéral (22) et le condyle fémoral médian (20) est plus petite que la somme du rayon de courbure de condyle fémoral latéral (38) et du rayon de courbure de condyle fémoral médian (36).

7. Endoprothèse de l'articulation du genou selon l'une des revendications précédentes, **caractérisée**
a) **en ce que** les points centraux (32, 34) de lignes d'intersection entre la au moins une surface d'articulation de ménisque (156, 158) et des plans d'intersection quelconques contenant l'axe de rotation (78) sont situés sur une surface cylindrique concentrique à l'axe de rotation (78)
et/ou
b) **en ce que** les points centraux (32, 34) sont situés dans un plan de points centraux qui s'étend transversalement, en particulier perpendiculairement, à l'axe de rotation (78)
et/ou
c) **en ce que** la partie ménisque (16) est agencée d'une pièce
et/ou
d) **en ce que** le dispositif de liaison (18) définit une position de liaison dans laquelle la partie tibia (14) et la partie fémur (12) sont reliées l'une à l'autre de manière inamovible
et/ou
e) **en ce que** la partie tibia (14) présente une surface d'appui de partie ménisque (98) tournée vers la partie ménisque (16) et en ce que l'axe de rotation (78) s'étend transversalement, en particulier perpendiculairement, à la surface d'appui de partie ménisque (98).

8. Endoprothèse de l'articulation du genou selon l'une des revendications précédentes, **caractérisée**
a) **en ce que** l'axe de charnière (66) s'étend perpendiculairement à l'axe de rotation (78)
et/ou
b) **en ce que** la partie tibia (14) présente une surface de tibia (98) sur laquelle s'appuie la partie ménisque (16) par un côté inférieur de partie ménisque (148), en particulier de manière plane, et en ce que l'axe de charnière (66) s'étend parallèlement ou sensiblement parallèlement à la surface de tibia (98).

9. Endoprothèse de l'articulation du genou selon l'une des revendications précédentes, **caractérisée**
a) **en ce que** l'axe de rotation (78) et l'axe de charnière (66) s'étendent de manière inclinée l'un par rapport à l'autre
et/ou
b) **en ce que** l'axe de rotation (78) est situé perpendiculairement à un plan contenant l'axe de charnière (66)
et/ou
c) **en ce que** le dispositif de liaison (18) est agencé de telle manière que la partie tibia (14) et la partie fémur (12) peuvent être reliées l'une à l'autre après une implantation sur le tibia et sur le fémur,
où en particulier un élément de palier rotatif (84) est agencé, formé ou maintenu sur la partie tibia (14) pour le montage de la partie fémur (12) de manière à pouvoir tourner autour de l'axe de rotation (78).

10. Endoprothèse de l'articulation du genou selon la revendication 9, **caractérisée**
a) **en ce que** l'élément de palier rotatif (84) est monté sur la partie tibia (14) de manière à pouvoir tourner autour de l'axe de rotation (78)
et/ou
b) **en ce que** la partie tibia (14) comprend un logement d'élément de palier rotatif (198) dans lequel l'élément de palier rotatif (84) est maintenu.

11. Endoprothèse de l'articulation du genou selon la revendication 10, **caractérisée**
a) **en ce que** l'élément de palier rotatif (84) est monté dans le logement d'élément de palier rotatif (198) de manière non déplaçable dans une direction parallèle à l'axe de rotation (78)
ou
**en ce que** l'élément de palier rotatif (84) est monté dans le logement d'élément de palier rotatif (198) de manière déplaçable dans une direction parallèle à l'axe de rotation (78)
et/ou
b) **en ce que** l'endoprothèse de l'articulation du genou comprend un dispositif de butée (200) pour limiter un mouvement de l'élément de palier rotatif (84) et du logement d'élément de palier rotatif (198) l'un par rapport à l'autre en direction distale et/ou proximale
et/ou
c) **en ce que** l'endoprothèse de l'articulation du genou comprend un dispositif de blocage (202) pour le blocage de l'élément de palier rotatif (84) dans le logement d'élément de palier rotatif (198)
et/ou
d) **en ce que** l'endoprothèse de l'articulation du genou comprend une douille de palier (134) pour le montage de l'élément de palier rotatif (84) qui définit le logement d'élément de palier rotatif (198).

12. Endoprothèse de l'articulation du genou selon la revendication 11, **caractérisée en ce que** la partie tibia (14) présente un logement de douille de palier (204) pour le logement de la douille de palier (134).

13. Endoprothèse de l'articulation du genou selon la revendication 12, **caractérisée**
a) **en ce que** le dispositif de blocage (202) comprend un élément de blocage (206) pour le blocage de la douille de palier (134) dans le logement de douille de palier (204)
et/ou
b) **en ce que** le dispositif de butée (200) comprend une butée qui limite une profondeur d'insertion de la douille de palier (134) dans le logement de douille de palier (204).

14. Endoprothèse de l'articulation du genou selon l'une des revendications 10 à 13, **caractérisée**
a) **en ce que** le logement d'élément de palier rotatif (198) est fermé en direction distale et ouvert en direction proximale
et/ou
b) **en ce que** le logement d'élément de palier rotatif (198) s'étend au moins en partie au-dessus et/ou en-dessous de la surface de tibia (98).

15. Endoprothèse de l'articulation du genou selon l'une des revendications précédentes, **caractérisée en ce que** la partie ménisque (16) est fabriquée en totalité ou en partie en une matière synthétique,
où en particulier la matière synthétique est ou comprend un polyéthylène (PE), un polyéthylène à ultrahaute masse moléculaire (UHMWPE) ou une polyétheréthercétone.
